# EUROPÄISCHE PATENTANMELDUNG

(11) **EP 1 125 587 A1**
(43) Veröffentlichungstag der Anmeldung: **22.08.2001**
(21) Anmeldenummer: 01890039.9
(22) Anmeldetag: 15.02.2001
(51) Int. Cl.: A61L 2/18, A61L 2/24, A61B 19/00, B08B 1/00, B08B 1/04

(54) **Vorrichtung zur mechanischen Reinigung und chemischen Desinfektion von medizinischen Instrumenten**

(30) Priorität: 15.02.2000 AT 2292000
(71) Anmelder: Ing. Knetsch, 1160 Wien (AT)
(72) Erfinder: Knetsch, Wilfried, Ing., 3013 Tullnerbach (AT)
(74) Vertreter: Müllner, Erwin, Dr.

(57) **Zusammenfassung**

Vorrichtung zur mechanischen Reinigung und chemischen Desinfektion von medizinischen Instrumenten (1), insbesondere von rotierenden zahnärztlichen Instrumenten, wie Turbinenwinkelstücken, Handstücken, Winkelstücken. Zur mechanischen Reinigung ist zumindest eine sich bewegende, von einem Motor (4) angetriebene Bürste (3), vorzugsweise eine Topfbürste, vorgesehen. Zur chemischen Desinfektion ist eine Sprühvorrichtung bestehend aus einer Einspritzdüse (5) und einer Dosierpumpe (6) zum Aufsprühen von Desinfektionsmittel vorgesehen.

## Beschreibung

Die vorliegende Erfindung betrifft eine Vorrichtung zur mechanischen Reinigung und chemischen Desinfektion von medizinischen Instrumenten, insbesondere von rotierenden zahnärztlichen Instrumenten, wie Turbinenwinkelstücken, Handstücken, Winkelstücken.

Die moderne Praxis-Hygiene verlangt, dass jedes Instrument keimfrei gemacht wird, bevor es mit dem Patienten in Berührung kommt. Hierfür gibt es derzeit in der Praxis verschiedene Möglichkeiten:
1. Sterilisation:
   Wird im Autoclav oder Chemiclave durchgeführt. Vorteil: absolute Keimfreiheit. Nachteil: übermäßige Materialbelastung (Kugellager), lange Sterilisationsdauer und damit erforderliche große Stückzahl an Instrumenten.
   Wird nur bei operativen Eingriffen angewandt.
2. Oberflächendesinfektion:
   Hierfür gibt es derzeit 2 Möglichkeiten:
   a) Das Instrument wird vor Gebrauch mit einem mit Desinfektionsmittel angefeuchteten Tuch abgewischt. Diese Methode wird überwiegend angewendet. Nachteile: relativ hoher Zeitaufwand, für den Patienten wenig sichtbar.
   b) Behandlung mit Sprüh-Desinfektion: ein Schweizer Gerät besprüht das eingeführte Instrument mit einem feinen Desinfektionsnebel. Vorteil: für den Patienten sehr eindrucksvolle Handhabung. Nachteil: Trotz Absaugung mit Kohlefilter starke Geruchsbelästigung, Instrument zu nass (Flüssigkeitsmenge nicht reduzierbar), Gerät sehr groß und teuer, teure Desinfektionsmittel.

Aus der US 5471706 ist eine Vorrichtung zur mechanischen Reinigung und chemischen Desinfektion von medizinischen Instrumenten bekannt, welches im Wesentlichen aus einem aufrecht stehenden Zylinder besteht, wobei von der Wand des Zylinders Borsten radial nach innen ragen. Im unteren Teil des Zylinders ist Desinfektionsmittel eingefüllt. Das zu reinigende Instrument wird von oben eingeführt, bis es in das Desinfektionsmittel eintaucht. Dabei muss es an allen Borsten vorbei, so dass es mechanisch gereinigt wird. Das Instrument wird dann auf und ab bewegt, damit es weiter über die Borsten gleitet und immer wieder in das Desinfektionsmittel eintaucht, so dass es gründlich gereinigt wird.

Nachteilig ist dabei, dass während der gesamten Dauer der Desinfektion eine Arbeitskraft damit beschäftigt ist, das zu reinigende Instrument hin und her zu bewegen.

Es ist Aufgabe der vorliegenden Erfindung, eine Vorrichtung zu schaffen, bei der die Reinigung (mechanisch durch Bürsten und chemisch durch Desinfektionsmittel) automatisch erfolgt.

Diese Aufgabe wird durch eine Vorrichtung der eingangs genannten Art erfindungsgemäß dadurch gelöst, dass zur mechanischen Reinigung zumindest eine sich bewegende, von einem Motor angetriebene Bürste vorgesehen ist und dass zur chemischen Desinfektion eine Sprühvorrichtung zum Aufsprühen von Desinfektionsmittel vorgesehen ist.

Vorzugsweise ist die Bürste eine Topfbürste.

Unter Topfbürste wird ein Zylinder mit den radial nach innen stehenden Borsten verstanden. Es ist also die Topfbürste um ihre Längsachse drehbar und z.B. von einem Getriebemotor antreibbar. Bei Einführen des Instrumentes wird von der Sprühvorrichtung Desinfektionsmittel eingespritzt und der Motor eingeschaltet. Rotationsdauer und Einspritzmenge sind justierbar. Das Desinfektionsmittel fließt dann in einen Entsorgungsbehälter.

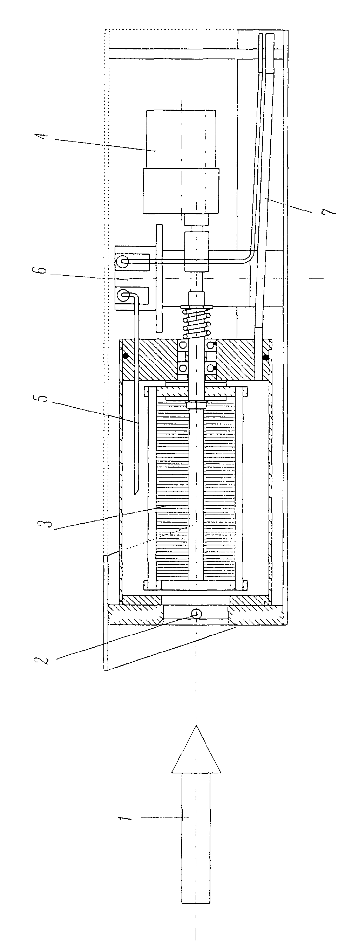

Anhand der beiliegenden Zeichnung wird die vorliegende Erfindung näher erläutert.

Das Gerät besteht aus zwei Baugruppen (Arbeitsmodul und zusammengefasste Module für Versorgung, Vorrat und Entsorgung). Beide Baugruppen können gemeinsam (als Tischgerät) oder getrennt (Unit-Montage) geliefert werden.

Im Arbeitsmodul befindet sich eine als Topfbürste ausgebildete Bürste 3, die von einem als Getriebemotor ausgeführten Motor 4 in Rotation versetzt werden kann. Über eine Einspritzdüse 5 kann von einer Dosierpumpe 6 durch die durchlässige Zylinderwand hindurch Desinfektionsmittel eingespritzt werden.

Die Vorrichtung wird wie folgt verwendet: Das Arbeitsmodul des Gerätes wird in Griffnähe des Zahnarztes und im Sichtfeld des Patienten montiert. Vor dem Aufstecken eines Instrumentes auf den Mikromotor oder auf den Turbinenschlauch der Dentaleinheit führt der Zahnarzt das Instrument 1 (durch einen Pfeil angedeutet) in das Arbeitsmodul der Vorrichtung ein. Dies wird von einer Lichtschranke 2 erkannt. Dadurch werden der Motor 4 und die Dosierpumpe 6 eingeschaltet. Durch die Einspritzdüse 5 wird eine fest eingestellte Zeit lang eine definierte Menge Desinfektionsmittel von außen auf die rotierende Bürste 3 gespritzt. Die Rotation der Bürste 3 erfolgt solange, wie das Instrument 1 die Lichtschranke 2 unterbricht. Das Desinfektionsmittel fließt anschließend über eine Abflussleitung 7 in einen Entsorgungsbehälter des Versorgungsmodules. In diesem Modul befindet sich auch der Desinfektionsmittel-Vorrat.

Die Vorteile sind: geringere Abmessungen, bei Aufsplittung einfachere Montage, keine Geruchsbelästigung, kostengünstiger Betrieb durch freie Wahl wasserlöslicher Desinfektionsmittel, zusätzliche mechanische Reinigung durch sechs Bürstenreihen, für den Patienten deutlich sichtbare Anwendung.

## Patentansprüche

1. Vorrichtung zur mechanischen Reinigung und chemischen Desinfektion von medizinischen Instrumenten, insbesondere von rotierenden zahnärztlichen Instrumenten, wie Turbinenwinkelstücken, Handstücken, Winkelstücken, **dadurch gekennzeichnet,** dass zur mechanischen Reinigung zumindest eine sich bewegende, von einem Motor (4) angetriebene Bürste (3) vorgesehen ist und dass zur chemischen Desinfektion eine Sprühvorrichtung (Einspritzdüse 5, Dosierpumpe 6) zum Aufsprühen von Desinfektionsmittel vorgesehen ist.

2. Vorrichtung nach Anspruch 1, **dadurch gekennzeichnet,** dass die Bürste (3) eine Topfbürste ist.
